# EUROPEAN PATENT SPECIFICATION

(11) **EP 3 380 169 B1**
(45) Date of publication and mention of the grant of the patent: **18.09.2019**
(21) Application number: 16798723.9
(22) Date of filing: 21.11.2016
(51) Int. Cl.: A61M 5/32

(54) **SYSTEM FOR CAP REMOVAL**
SYSTEM ZUR KAPPENENTFERNUNG
SYSTÈME POUR LE RETRAIT D'UN CAPUCHON

(30) Priority: 27.11.2015 EP 15196682
(43) Date of publication of application: 03.10.2018
(73) Proprietor: Sanofi-Aventis Deutschland GmbH, 65929 Frankfurt am Main (DE)
(72) Inventor: WENDLAND, Stefan, 65926 Frankfurt am Main (DE); HARMS, Michael, 65926 Frankfurt am Main (DE)
(74) Representative: Brown, Alexander Edward
(86) International application number: PCT/EP2016/078253
(87) International publication number: WO 2017/089264

(56) References cited:
- EP-A1- 2 420 267
- WO-A1-2008/086004
- WO-A1-2009/081133
- GB-A- 2 518 646
- US-A- 5 224 936

## Description

This application relates to an injector drug delivery device. Injector devices have application where regular injections by persons without formal medical training occur. This is common among patients where self-treatment enables effective management of their disease.

Such devices are used to deliver a range of liquid medicaments. Certain medicaments, such as insulin, require storing in a refrigerator in order to retain efficacy. It is advisable when injecting a refrigerated medicament to allow the medicament to warm to room temperature. This reduces the discomfort of the injection process, maximises the efficacy of the medicament and reduces the force required to dispense the medicament through the needle.

Injector devices comprise caps to protect the needle and prevent accidental injury of the user by the needle. The cap must therefore be firmly attached to the injector device to prevent premature removal of the cap. However, this means that removal of the cap by elderly or physically infirm patients may be difficult.

It is an object of the invention to address the problems mentioned above and provide an improved injector device.

Reference is made to US5224936, which discloses an automatic self-protecting hypodermic needle assembly.

According to the invention there is provided an auto-injector device for delivering a liquid medicament, comprising an injector body, a syringe received in the injector body, a needle disposed in a first end of the syringe to extend toward an opening of the injector body, an injector cap to enclose the opening and cover the needle, and a releasable attachment mechanism configured to secure the cap in place covering the needle and comprising first and second engaging parts, wherein the first and second engaging parts cooperate in an attached position to secure the cap in position covering the needle and wherein the attachment mechanism is configured to move into a release position in which the first and second engaging parts are in reduced cooperation when above a threshold temperature to facilitate detachment of the injector cap to expose the needle.

It is intended that the injector device is stored in a refrigerator at a temperature less than the threshold temperature. Therefore, with the device stored in the refrigerator, the cap is securely retained on the device by the attachment mechanism. With the device removed from the refrigerator, where the temperature exceeds the threshold temperature, the attachment mechanism is moved to the release position so that the cap is easily detached.

At least one of the first and second engaging parts may be biased out of engagement with the other of the first and second engaging parts into the release position when above the threshold temperature.

Therefore the cap can be freely or more easily removed from the device so that the cap is removable by an elderly or physically impaired patient.

The first engaging part may be provided on the cap and second engaging part may be provided on the body or the syringe wherein the first and second engaging parts cooperate to secure the cap to the body or the syringe in the attached position, and wherein the first and or second engaging part is biased into the release position when said first and or second engaging part is above the threshold temperature so as to facilitate detachment of the injector cap from the body and or the syringe.

Therefore the cap cooperates with the body or the syringe to secure the cap thereon when the device is below the threshold temperature.

The cap may comprise a needle shield that extends into the injector body to enclose the needle and wherein the first engaging part is a distal end of the needle shield and the second engaging part is the first end of the syringe.

Therefore the needle is protected from contaminants by the needle shield.

The needle shield may be formed of a I material with thermomechanical properties (hereinafter referred to as thermomechanical material) such that, below the threshold temperature the needle shield has a first diameter in which the needle shield abuts the first end of the syringe and wherein, above the threshold temperature, the needle shield is biased into the release position in which the needle shield has a second diameter to be spaced from the first end of the syringe.

Therefore the needle shield is held on the syringe by frictional engagement of the needle shield when the syringe is below the threshold temperature.

The cap may have an outer wall that extends over an outer surface of the body and wherein the first engaging part is a clip formed in the outer wall and the second engaging part is an opening formed in the outer surface of the injector body.

Therefore the cap is held on the body by the clip so that the cap cannot be removed at a temperature below the threshold temperature.

The clip may be formed of a thermomechanical material such that, below the threshold temperature, the clip is disposed in the opening in the outer surface of the injector body and wherein, above the threshold temperature, the clip is biased away from the outer surface of the injector body into the release position, wherein the clip is removed from the opening.

Therefore, above the threshold temperature, the cap can be freely removed from the device so that the cap is removable by an elderly or physically impaired patient.

The cap may have an outer wall that extends over an outer surface of the body and wherein the first engaging part is an opening formed through the outer wall, and wherein the second engaging part comprises a clip formed in the injector body.

Therefore the cap is held on the body by the clip at a temperature below the threshold temperature. Advantageously, the cap can be removed from the body at a temperature below the threshold temperature by manually displacing the clip through the opening in the cap.

The second engaging part may comprise a cantilever spring having a fixed end depending from an internal surface of the injector body and a free end mechanically coupled to the clip.
The cantilever spring may be formed of a thermomechanical material such that at, below the threshold temperature, the spring is configured to bias the clip so that it is disposed in the opening and wherein, above the threshold temperature, the spring is configured to bias the clip into the release position, wherein the clip is removed from the opening.

Therefore the thermomechanical material is not the clip.

The thermomechanical material may be a bimetallic material.

The thermomechanical material may be a shape memory alloy.

According to the invention there is provided an auto-injector device as described above comprising a cartridge of liquid medicament.

So that the present invention may be more fully understood embodiments thereof will now be described with reference to the accompanying drawings in which:
Figure 1A shows an auto injector with a cap attached;
Figure 1B shows the auto injector of Figure 1A with the cap removed;
Figure 2A shows a partial view of an auto-injector with the cap attached according to a first embodiment of the invention;
Figure 2B shows a partial view of the auto-injector of Figure 2A with the cap released;
Figure 3 shows a partial view of an auto-injector according to a second embodiment of the invention;
Figure 4A shows a partial view of an auto-injector with the cap attached according to a third embodiment of the invention;
Figure 4B shows a partial view of the auto-injector of Figure 4A with the cap released;
Figure 4C shows a partial view of the auto-injector of Figure 4A with the cap removed.

A drug delivery device, as described herein, may be configured to inject a medicament into a patient. For example, delivery could be sub-cutaneous, intra-muscular, or intravenous. Such a device could be operated by a patient or care-giver, such as a nurse or physician, and can include various types of safety syringe, pen-injector, or auto-injector. The device can include a cartridge-based system that requires piercing a sealed ampule before use. Volumes of medicament delivered with these various devices can range from about 0.5 ml to about 2 ml. Yet another device can include a large volume device ("LVD") or patch pump, configured to adhere to a patient's skin for a period of time (e.g., about 5, 15, 30, 60, or 120 minutes) to deliver a "large" volume of medicament (typically about 2 ml to about 10 ml).

In combination with a specific medicament, the presently described devices may also be customized in order to operate within required specifications. For example, the device may be customized to inject a medicament within a certain time period (e.g., about 3 to about 20 seconds for auto-injectors, and about 10 minutes to about 60 minutes for an LVD). Other specifications can include a low or minimal level of discomfort, or to certain conditions related to human factors, shelf-life, expiry, biocompatibility, environmental considerations, etc. Such variations can arise due to various factors, such as, for example, a drug ranging in viscosity from about 3 cP to about 50 cP. Consequently, a drug delivery device will often include a hollow needle ranging from about 25 to about 31 Gauge in size. Common sizes are 27 and 29 Gauge.

The delivery devices described herein can also include one or more automated functions. For example, one or more of needle insertion, medicament injection, and needle retraction can be automated. Energy for one or more automation steps can be provided by one or more energy sources. Energy sources can include, for example, mechanical, pneumatic, chemical, or electrical energy. For example, mechanical energy sources can include springs, levers, elastomers, or other mechanical mechanisms to store or release energy. One or more energy sources can be combined into a single device. Devices can further include gears, valves, or other mechanisms to convert energy into movement of one or more components of a device.

The one or more automated functions of an auto-injector may each be activated via an activation mechanism. Such an activation mechanism can include one or more of a button, a lever, a needle sleeve, or other activation component. Activation of an automated function may be a one-step or multi-step process. That is, a user may need to activate one or more activation components in order to cause the automated function. For example, in a one-step process, a user may depress a needle sleeve against their body in order to cause injection of a medicament. Other devices may require a multi-step activation of an automated function. For example, a user may be required to depress a button and retract a needle shield in order to cause injection.

In addition, activation of one automated function may activate one or more subsequent automated functions, thereby forming an activation sequence. For example, activation of a first automated function may activate at least two of needle insertion, medicament injection, and needle retraction. Some devices may also require a specific sequence of steps to cause the one or more automated functions to occur. Other devices may operate with a sequence of independent steps.

Some delivery devices can include one or more functions of a safety syringe, pen-injector, or auto-injector. For example, a delivery device could include a mechanical energy source configured to automatically inject a medicament (as typically found in an auto-injector) and a dose setting mechanism (as typically found in a pen-injector).

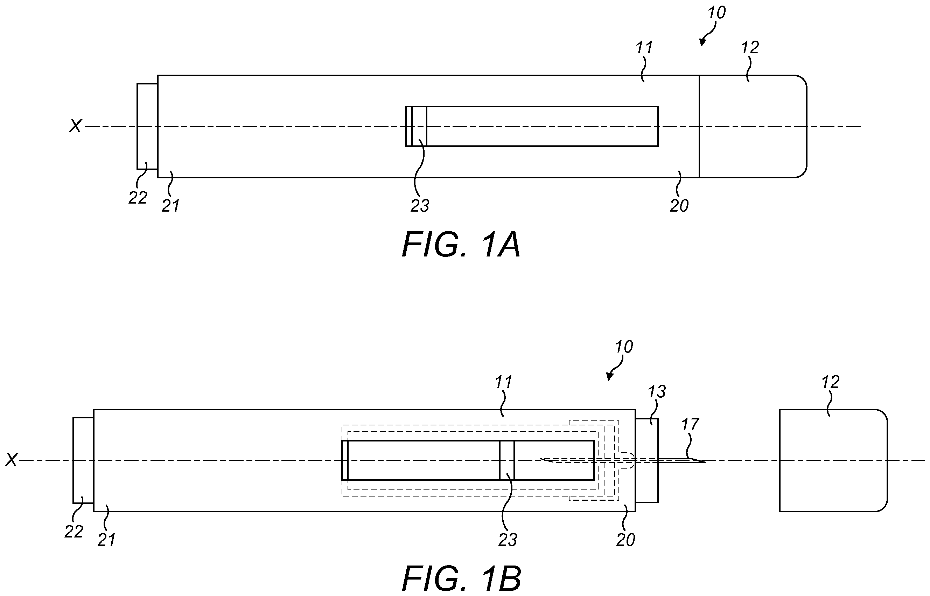

According to some embodiments of the present disclosure, an exemplary drug delivery device 10 is shown in Figs. 1A & 1B. Device 10, as described above, is configured to inject a medicament into a patient's body. Device 10 includes a housing 11 which typically contains a reservoir containing the medicament to be injected (e.g., a syringe) and the components required to facilitate one or more steps of the delivery process. Device 10 can also include a cap assembly 12 that can be detachably mounted to the housing 11. Typically a user must remove cap 12 from housing 11 before device 10 can be operated.

As shown, housing 11 is substantially cylindrical and has a substantially constant diameter along the longitudinal axis X. The housing 11 has a distal region 20 and a proximal region 21. The term "distal" refers to a location that is relatively closer to a site of injection, and the term "proximal" refers to a location that is relatively further away from the injection site.

Device 10 can also include a needle sleeve 13 coupled to housing 11 to permit movement of sleeve 13 relative to housing 11. For example, sleeve 13 can move in a longitudinal direction parallel to longitudinal axis X. Specifically, movement of sleeve 13 in a proximal direction can permit a needle 17 to extend from distal region 20 of housing 11.

Insertion of needle 17 can occur via several mechanisms. For example, needle 17 may be fixedly located relative to housing 11 and initially be located within an extended needle sleeve 13. Proximal movement of sleeve 13 by placing a distal end of sleeve 13 against a patient's body and moving housing 11 in a distal direction will uncover the distal end of needle 17. Such relative movement allows the distal end of needle 17 to extend into the patient's body. Such insertion is termed "manual" insertion as needle 17 is manually inserted via the patient's manual movement of housing 11 relative to sleeve 13.

Another form of insertion is "automated," whereby needle 17 moves relative to housing 11. Such insertion can be triggered by movement of sleeve 13 or by another form of activation, such as, for example, a button 22. As shown in Figs. 1A & 1B, button 22 is located at a proximal end of housing 11. However, in other embodiments, button 22 could be located on a side of housing 11.

Other manual or automated features can include drug injection or needle retraction, or both. Injection is the process by which a bung or piston 23 is moved from a proximal location within a syringe (not shown) to a more distal location within the syringe in order to force a medicament from the syringe through needle 17. In some embodiments, a drive spring (not shown) is under compression before device 10 is activated. A proximal end of the drive spring can be fixed within proximal region 21 of housing 11, and a distal end of the drive spring can be configured to apply a compressive force to a proximal surface of piston 23. Following activation, at least part of the energy stored in the drive spring can be applied to the proximal surface of piston 23. This compressive force can act on piston 23 to move it in a distal direction. Such distal movement acts to compress the liquid medicament within the syringe, forcing it out of needle 17.

Following injection, needle 17 can be retracted within sleeve 13 or housing 11. Retraction can occur when sleeve 13 moves distally as a user removes device 10 from a patient's body. This can occur as needle 17 remains fixedly located relative to housing 11. Once a distal end of sleeve 13 has moved past a distal end of needle 17, and needle 17 is covered, sleeve 13 can be locked. Such locking can include locking any proximal movement of sleeve 13 relative to housing 11.

Another form of needle retraction can occur if needle 17 is moved relative to housing 11. Such movement can occur if the syringe within housing 11 is moved in a proximal direction relative to housing 11. This proximal movement can be achieved by using a retraction spring (not shown), located in distal region 20. A compressed retraction spring, when activated, can supply sufficient force to the syringe to move it in a proximal direction. Following sufficient retraction, any relative movement between needle 17 and housing 11 can be locked with a locking mechanism. In addition, button 22 or other components of device 10 can be locked as required.

According to embodiments of the invention, the auto injector 10 comprises first and second engaging parts that cooperate to retain the cap 12 in the attached position. The engaging parts are configured to move into a release position to release the cap 12 when the respective parts reach a threshold temperature. With the cap 12 released, the cap 12 can be freely or more easily removed to expose the needle 17 so that the user can inject the medicament 16.

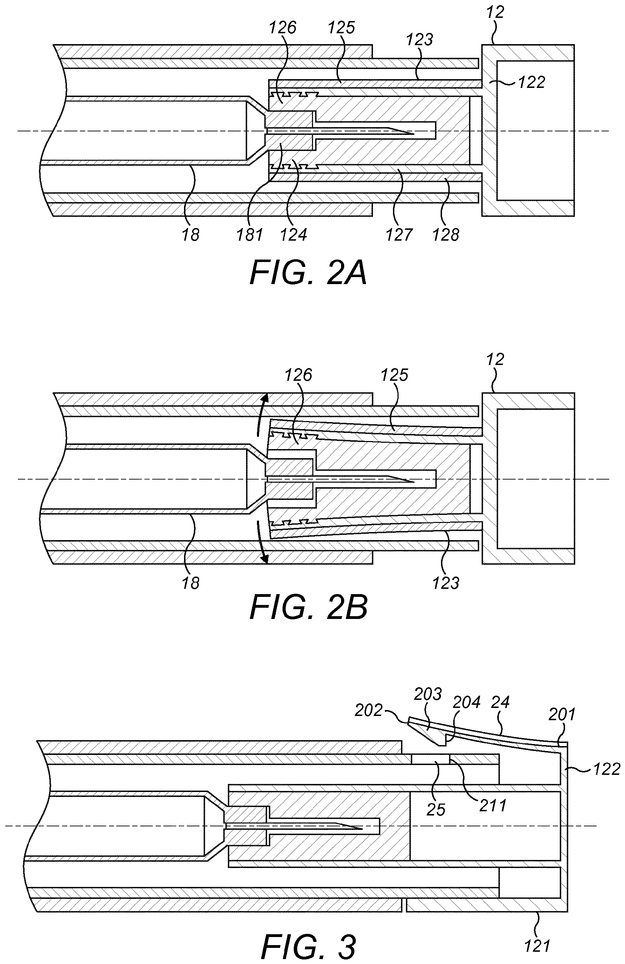

Referring now to Figures 2A and 2B, an auto injector 10 is shown according to a first embodiment of the invention. In Figure 2A the cap of the auto injector is shown in an attached position. The cap 12 comprises a cylindrical needle shield 123 that extends from the end wall 122 of the cap 12 into the injector body to enclose the needle 17. The first engaging part is an end portion 124 of the needle shield 123 and the second engaging part is the distal end 181 of the syringe 18. An internal surface of the end portion 124 of the needle shield 123 tightly abuts an external surface of the distal end 181 of the syringe 18 to retain the cap 12 thereon.

The needle shield 123 comprises an outer structure 125 and an inner structure 126. The inner structure 126 lines the outer structure 125 and forms the internal surface of the needle shield 123. The inner structure 126 is permanently attached to the outer structure 125 by adhesive, interference fit, or other mechanical means. The inner structure 126 is made from a material of high coefficient of friction such as rubber or similar. Therefore, with the cap 12 in the attached position and the internal surface of the needle shield 123 tightly abutting the external surface of the syringe 18, the cap 12 is retained on the syringe 18 by frictional engagement of the respective internal and external surfaces. With the cap 12 thus attached, a substantial force is required to remove the cap 12 from the syringe 18.

The outer structure 125 is made from a bimetallic material. The bi-metallic material is configured to change shape as the temperature of the material changes to release the cap 12 so that the cap 12 can be more easily removed from the syringe 18.

The bimetallic outer structure 125 is formed of two metal layers, each having a different coefficient of thermal expansion. A first layer 127 is disposed between a second layer 128 and the inner structure 126. The first layer 127 has a greater coefficient of thermal expansion than the second layer 128 so that, as the temperature of the outer structure 125 increases, the first layer 127 expands at a greater rate than the second layer 128 to cause the outer structure 125 and the inner structure 126 to deform away from the syringe 18. When the outer structure 125 reaches a threshold temperature the inner surface of the needle shield 123 no longer abuts the external surface of the syringe 18, as shown in Figure 2B, such that the cap 12 is in a release position in which it is no longer retained on the syringe 18 by frictional engagement of the respective surfaces, thus making the cap 12 easier to remove.

It shall be appreciated that an outer structure 125 made from a bimetallic material represents a preferred embodiment only, and that the outer structure 125 may be formed of any material having a greater coefficient of thermal expansion than the distal end 181 of the syringe 18. In this way, as the needle shield 123 and the syringe 18 increase in temperature, the needle shield 123 expands at a greater rate than the distal end 181 of the syringe 18 to reduce the compressive contact of the inner surface of the needle shield 123 against the external surface of the syringe 18. In this example of the first embodiment, the reduced compressive contact of the respective internal and external surfaces results in reduced frictional engagement of said surfaces so that the cap 12 is easier to remove.

It is envisaged that the threshold temperature is any temperature between 15 and 25 Celsius. Therefore, the threshold temperature advantageously corresponds to a temperature at which the medicament 16 reaches a comfortable injecting temperature so that the cap 12 is more easily or freely removed when the medicament 16 has reached a comfortable injecting temperature. The threshold temperature may also correspond to a temperature which maximises the efficacy of the medicament 16 or even a temperature at which the medicament 16 is more easily dispensed from the syringe.

It is envisaged that the medicament will be stored at a temperature between -5 and 5 Celsius. According to the invention the bi metallic strip is configured so that the first and second engaging parts are engaged in this temperature range. The bimetallic strip is further configured to displace the first and second engaging parts apart a distance of 5mm for an increase in temperature of 10 degrees Celsius. Therefore, a minimum temperature increase of 10 Celsius, from 5 Celsius to 15 Celsius, causes a 5mm displacement apart of the first and second engaging parts to allow the cap 12 to be freely or easily removed.

According to embodiments of the invention, a thermochromic strip (not shown) may be provided on the cap 12. The thermochromic strip is provided to indicate that the threshold temperature has been reached. The thermochromic strip is provided on the outer surface of the cap 12 so that it is visible to the user. The thermochromic strip is configured to change colour so that an indicating portion of the thermochromic strip is illuminated when the threshold temperature has been reached. Therefore the user is able to quickly and easily identify when the cap 12 is ready to be freely or easily removed.

A second embodiment of the invention is shown in Figure 3 in which features in common with the first embodiment retain the same reference numerals. In the second embodiment, the first engaging part is a clip 24 provided in the cylindrical wall 121 of the cap 12 and the second engaging part is an opening 25 provided in the external portion of the sleeve 13. The clip 24 retains the cap 12 in the attached position when the clip 24 is below the threshold temperature.

The clip 24 is delimited by two cut lines that extend in parallel from the open end of the cylindrical wall 121 toward the end wall 122 of the cap 12. The cut lines disconnect the clip 24 from the remainder of the cylindrical wall 121 so that the clip 24 is displaceable relative to the cylindrical wall 121. The clip 24 has a fixed end 201 where the cut lines terminate and a free end 202 that cantilevers from the fixed end 201. An engagement tab 203 extends from the free end 202 of the clip 24 and comprises an engaging surface 204 disposed normal to the internal surface of the cylindrical wall 121.

With the cap 12 in an attached position, the engagement tab 203 extends through the opening 25 provided in the sleeve 13. The engaging surface 204 abuts a distal edge 211 of the opening 25 to prevent axial translation of the cap 12. The clip 24 is formed of a bimetallic material having a first layer and a second layer in which the first layer has a greater coefficient of thermal expansion. As the temperature of the clip 24 increases, the first layer expands at a greater rate than the second layer to cause the clip 24 to deflect away from the external surface of the external portion of the sleeve 13. When the clip 24 reaches a threshold temperature, the attachment tab 203 is deflected clear of the opening 25 to release the cap 12 so that the cap 12 is in a release position and can be removed from the sleeve 13.

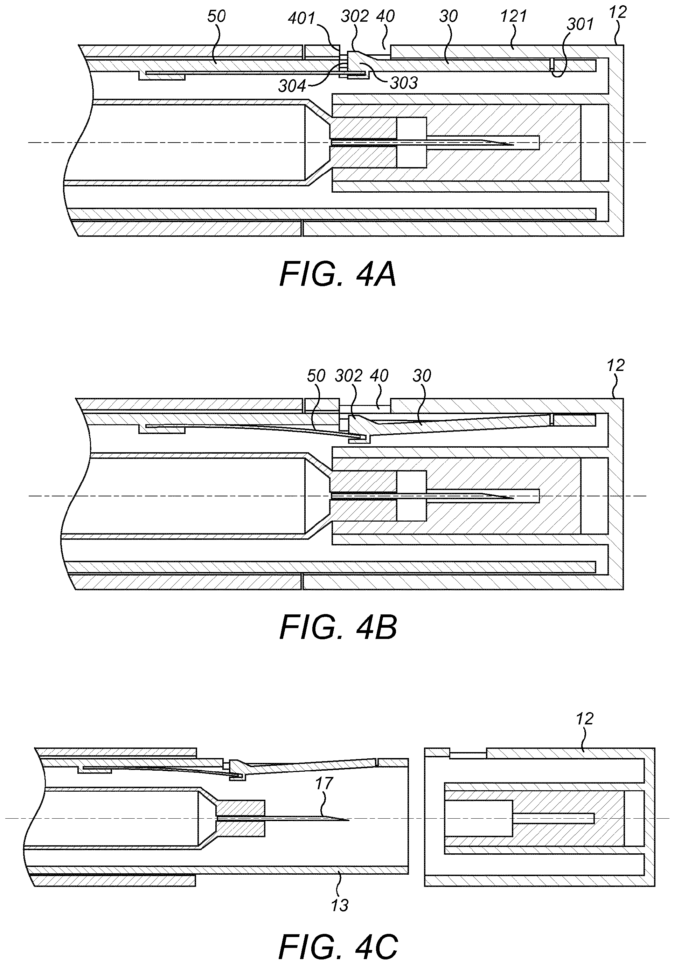

A third embodiment of the invention is shown Figures 4A to 4C. In this third embodiment the second engaging part is a clip 30 provided in the external portion of the sleeve 13 and the first engaging part is an opening 40 provided in the cylindrical wall 121 of the cap 12. As with the second embodiment of the invention described above, the clip 30 prevents the cap 12 from being removed when the clip 30 is below the threshold temperature.

The clip 30 is delimited by two cut lines that extend in parallel along a section of the sleeve 13. The cut lines disconnect the clip 30 from the remainder of the sleeve 13 so that the clip 30 is displaceable relative to the sleeve 13. The clip 30 has a fixed end 301 where the cut lines terminate and a free end 302 that cantilevers from the fixed end 301. An engagement tab 303 extends from the free end 302 of the clip 30 and comprises an engaging surface 304 disposed normal to the external surface of the sleeve 13.

With the cap 12 in an attached position as shown in Figure 4A, the engagement tab 303 extends through the opening 40 provided in the cap 12. The engaging surface 304 abuts a proximal edge 401 of the opening 40 to prevent axial translation of the cap 12.

According to the third embodiment, a cantilever metal spring 50 depends from an internal surface of the sleeve 13 to extend from the internal surface of the sleeve 13 into engagement with the free end 302 of the clip 30.

The cantilever spring 50 is made from a bimetallic material with two layers, in which the first layer has a greater coefficient of thermal expansion. As the temperature of the spring 50 increases, the first layer expands at a greater rate to cause the spring 50 to deflect away from the internal surface of the cylindrical wall 121 of the cap 12. The spring 50 is mechanically coupled to the clip 30 by engagement with the free end 302 of the clip 30 so that, as the spring 50 is caused to deflect by an increase in temperature, the free end 302 of the clip 30 is biased away from internal surface of the cylindrical wall 121 of the cap 12 by the spring 50. When the spring 50 reaches the threshold temperature, the free end 302 of the clip 30 is deflected clear of the opening 40 by the spring 50 so that the cap 12 is in a release position, as shown in Figure 4B, so that the cap 12 can be removed from the sleeve 13 to uncover the needle 17, as shown in Figure 4C.

Although in above embodiments the inner structure 126 of the needle shield 123 is made from a high friction material, it shall be appreciated that this is a preferential embodiment only. Other materials such as plastics could be used. For example, in one embodiment the inner structure 126 may be formed integrally with the end wall 122 of the cap 12 with the bimetallic material bonded as a strip to the outer surface of the inner structure 126.

In the above embodiments, the cylindrical wall 121 of the cap 12 and the external surface of the sleeve 13 only lightly abut so as to limit the frictional engagement of the respective surfaces. Therefore, when the cap 12 is released by the respective engaging parts, the cap 12 is easily removed from the sleeve 13 so that the device is more easily handled by elderly users, or users with a physical impairment.

In the first embodiment, the cylindrical wall 121 of the cap 12 is optional and may be omitted, as illustrated in Figures 2A and 2B.

In yet another embodiment, the internal surface of cylindrical wall 121 of the cap 12 tightly abuts an external surface of the sleeve 13 to hold the cap 12 in the attached position. In this embodiment, the cylindrical wall 121 of the cap 12 comprises a bimetallic layer so that, as the temperature of the bimetallic layer increases, the cylindrical wall 121 of the cap 12 deforms away from the external surface of the sleeve 13, such that the cap 12 is in a release position in which it is no longer retained on the sleeve 13 by the frictional engagement of the respective surfaces, thus making the cap 12 easier to remove.

Examples of bimetallic materials as used in the above embodiments can include TB20110 or TB 1577A when defined according to DIN 1715. However, it shall be appreciated that any suitable bimetallic material can be used. Furthermore, where a bimetallic material has been used, other temperature sensitive materials may be substitutable, for example certain shape memory alloys such as NiTi, Cu-Zn-Al and Cu-Al-Ni all have the requisite temperature sensitive properties.

Those of skill in the art will understand that modifications (additions and/or removals) of various components of the substances, formulations, apparatuses, methods, systems and embodiments described herein may be made without departing from the full scope and spirit of the present invention, which encompass such modifications and any and all equivalents thereof.

The terms "drug" or "medicament" are used herein to describe one or more pharmaceutically active compounds. As described below, a drug or medicament can include at least one small or large molecule, or combinations thereof, in various types of formulations, for the treatment of one or more diseases. Exemplary pharmaceutically active compounds may include small molecules; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more of these drugs are also contemplated. The term "drug delivery device" shall encompass any type of device or system configured to dispense a drug into a human or animal body. Without limitation, a drug delivery device may be an injection device (e.g., syringe, pen injector, auto injector, large-volume device, pump, perfusion system, or other device configured for intraocular, subcutaneous, intramuscular, or intravascular delivery), skin patch (e.g., osmotic, chemical, micro-needle), inhaler (e.g., nasal or pulmonary), implantable (e.g., coated stent, capsule), or feeding systems for the gastrointestinal tract. The presently described drugs may be particularly useful with injection devices that include a needle, e.g., a small gauge needle.

The drug or medicament may be contained in a primary package or "drug container" adapted for use with a drug delivery device. The drug container may be, e.g., a cartridge, syringe, reservoir, or other vessel configured to provide a suitable chamber for storage (e.g., short- or long-term storage) of one or more pharmaceutically active compounds. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber cartridge configured to store two or more components of a drug formulation (e.g., a drug and a diluent, or two different types of drugs) separately, one in each chamber. In such instances, the two chambers of the dual-chamber cartridge may be configured to allow mixing between the two or more components of the drug or medicament prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.

The drug delivery devices and drugs described herein can be used for the treatment and/or prophylaxis of many different types of disorders. Exemplary disorders include, e.g., diabetes mellitus or complications associated with diabetes mellitus such as diabetic retinopathy, thromboembolism disorders such as deep vein or pulmonary thromboembolism. Further exemplary disorders are acute coronary syndrome (ACS), angina, myocardial infarction, cancer, macular degeneration, inflammation, hay fever, atherosclerosis and/or rheumatoid arthritis. Exemplary drugs for the treatment and/or prophylaxis of diabetes mellitus or complications associated with diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the term "derivative" refers to any substance which is sufficiently structurally similar to the original substance so as to have substantially similar functionality or activity (e.g., therapeutic effectiveness).

Exemplary insulin analogues are Gly(A21), Arg(B31), Arg(B32) human insulin (insulin glargine); Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.
Exemplary insulin derivatives are, for example, B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-gamma-glutamyl)-des(B30) human insulin; B29-N-(N-lithocholyl-gamma-glutamyl)-des(B30) human insulin; B29-N-(ω-carboxyheptadecanoyl)-des(B30) human insulin and B29-N-(ω-carboxyheptadecanoyl) human insulin. Exemplary GLP-1, GLP-1 analogues and GLP-1 receptor agonists are, for example: Lixisenatide / AVE0010 / ZP10 / Lyxumia, Exenatide / Exendin-4 / Byetta / Bydureon / ITCA 650 / AC-2993 (a 39 amino acid peptide which is produced by the salivary glands of the Gila monster), Liraglutide / Victoza, Semaglutide, Taspoglutide, Syncria / Albiglutide, Dulaglutide, rExendin-4, CJC-1134-PC, PB-1023, TTP-054, Langlenatide / HM-11260C, CM-3, GLP-1 Eligen, ORMD-0901, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, TT-401, BHM-034. MOD-6030, CAM-2036, DA-15864, ARI-2651, ARI-2255, Exenatide-XTEN and Glucagon-Xten.

An exemplary oligonucleotide is, for example: mipomersen / Kynamro, a cholesterol-reducing antisense therapeutic for the treatment of familial hypercholesterolemia.
Exemplary DPP4 inhibitors are Vildagliptin, Sitagliptin, Denagliptin, Saxagliptin, Berberine. Exemplary hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.
Exemplary polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 / Synvisc, a sodium hyaluronate.

The term "antibody", as used herein, refers to an immunoglobulin molecule or an antigen-binding portion thereof. Examples of antigen-binding portions of immunoglobulin molecules include F(ab) and F(ab')2 fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region.
The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present invention include, for example, Fab fragments, F(ab')2 fragments, scFv (single-chain Fv) fragments, linear antibodies, monospecific or multispecific antibody fragments such as bispecific, trispecific, and multispecific antibodies (e.g., diabodies, triabodies, tetrabodies), minibodies, chelating recombinant antibodies, tribodies or bibodies, intrabodies, nanobodies, small modular immunopharmaceuticals (SMIP), binding-domain immunoglobulin fusion proteins, camelized antibodies, and VHH containing antibodies. Additional examples of antigen-binding antibody fragments are known in the art.

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.
Exemplary antibodies are anti PCSK-9 mAb (e.g., Alirocumab), anti IL-6 mAb (e.g., Sarilumab), and anti IL-4 mAb (e.g., Dupilumab).

The compounds described herein may be used in pharmaceutical formulations comprising (a) the compound(s) or pharmaceutically acceptable salts thereof, and (b) a pharmaceutically acceptable carrier. The compounds may also be used in pharmaceutical formulations that include one or more other active pharmaceutical ingredients or in pharmaceutical formulations in which the present compound or a pharmaceutically acceptable salt thereof is the only active ingredient. Accordingly, the pharmaceutical formulations of the present disclosure encompass any formulation made by admixing a compound described herein and a pharmaceutically acceptable carrier.

Pharmaceutically acceptable salts of any drug described herein are also contemplated for use in drug delivery devices. Pharmaceutically acceptable salts are for example acid addition salts and basic salts. Acid addition salts are e.g. HCl or HBr salts. Basic salts are e.g. salts having a cation selected from an alkali or alkaline earth metal, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are known to those of skill in the arts.

Pharmaceutically acceptable solvates are for example hydrates or alkanolates such as methanolates or ethanolates.

Those of skill in the art will understand that modifications (additions and/or removals) of various components of the substances, formulations, apparatuses, methods, systems and embodiments described herein may be made without departing from the full scope of the present invention, which encompass such modifications and any and all equivalents thereof.

## Claims

1. An auto-injector device for delivering a liquid medicament, comprising:
- an injector body,
- a syringe(18) received in the injector body
- a needle (17) disposed in a first end (181) of the syringe (18) to extend toward an opening of the injector body,
- an injector cap (12) to enclose the opening and cover the needle, and
- a releasable attachment mechanism configured to secure the cap in place covering the needle (17) and comprising first and second engaging parts (124, 181; 24, 25; 40, 30, 50);
**characterized in that** the first and second engaging parts (124, 181; 24, 25; 40, 30, 50) cooperate in an attached position to secure the cap (12) in position covering the needle (17) and wherein the attachment mechanism is configured to move into a release position in which the first and second engaging parts (124, 181; 24, 25; 40, 30, 50) are in reduced cooperation when above a threshold temperature to facilitate detachment of the injector cap (12) to expose the needle (17).

2. An auto-injector according to claim 1 wherein at least one of the first and second engaging parts (124, 181; 24, 25; 40, 30, 50) is biased out of engagement with the other of the first and second engaging parts (124, 181; 24, 25; 40, 30, 50) into the release position when above the threshold temperature.

3. An auto injector according to claim 1 or claim 2 wherein the first engaging part (124, 24, 40) is provided on the cap (12) and second engaging part (181, 25, 30) is provided on the body or the syringe (18) and wherein the first and second engaging parts (124, 181; 24, 25; 40, 30, 50) cooperate to secure the cap (12) to the body or the syringe (18) in the attached position, and wherein the first and or second engaging part (124, 181; 24, 25; 40, 30, 50) is biased into the release position when said first and or second engaging part (124, 181; 24, 25; 40, 30, 50) is above the threshold temperature so as to facilitate detachment of the injector cap (12) from the body and or the syringe (18).

4. An auto injector according to any preceding claim, wherein the cap (12) comprises a needle shield (123) that extends into the injector body to enclose the needle (17) and wherein the first engaging part (124) is an open end of the needle shield (123) and the second engaging part (181) is the first end (181) of the syringe (18).

5. An auto injector according to claim 4, wherein the needle shield (123) is formed of a thermomechanical material such that below the threshold temperature the needle shield (123) has a first diameter in which the needle shield (123) abuts the first end (181) of the syringe (18), and wherein above the threshold temperature the needle shield (123) is biased into the release position in which the needle shield (123) has a second diameter to be spaced from the first end (181) of the syringe (18).

6. An auto injector according to any of claims 1 to 3, wherein the cap (12) has an outer wall (121) that extends over an outer surface of the body and wherein the first engaging part (24) is a clip (24) formed in the outer wall (121) and the second engaging part (25) is an opening (25) formed in the outer surface of the injector body.

7. An auto injector according to claim 6, wherein the clip (24) is formed of a thermomechanical material such that below the threshold temperature the clip (24) is disposed in the opening (25) in the outer surface of the injector body, and wherein above the threshold temperature the clip (24) is biased away from the outer surface of the injector body into the release position, wherein the clip (24) is removed from the opening (25).

8. An auto injector according to any of claims 1 to 3, wherein the cap (12) has an outer wall (121) that extends over an outer surface of the body and wherein the first engaging part (40) is an opening (40) formed through the outer wall (121), and wherein the second engaging part (30, 50) comprises a clip (30) formed in the injector body.

9. An auto injector according to claim 8, wherein the second engaging part (30, 50) further comprises a cantilever spring (50) having a fixed end depending from an internal surface of the injector body and a free end mechanically coupled to the clip (30).

10. An auto injector according to claim 9, wherein the cantilever spring (50) is formed of a thermomechanical material such that below the threshold temperature the spring (50) is configured to bias the clip (30) so that it is disposed in the opening (40), and wherein above the threshold temperature the spring (50) is configured to bias the clip (30) into the release position, wherein the clip (30) is removed from the opening.

11. An auto injector according to any of claims 5, 7 or 10, wherein the thermomechanical material is a bimetallic material.

12. An auto injector according to any of claims 5, 7 or 10, wherein the thermomechanical material is a shape memory alloy.

13. An auto injector device according to any preceding claim comprising a cartridge of liquid medicament.

## Patentansprüche

1. Autoinjektorvorrichtung zur Verabreichung eines flüssigen Medikaments, aufweisend:
- einen Injektorkörper,
- eine in dem Injektorkörper aufgenommene Spritze (18),
- eine Nadel (17), die in einem erste Ende (181) der Spritze (18) angeordnet ist, um sich zu einer Öffnung des Injektorkörpers hin zu erstrecken,
- eine Injektorkappe (12), um die Öffnung zu umschließen und die Nadel abzudecken, und
- einen freigebbaren Anbringmechanismus, der dazu ausgestaltet ist, die Kappe an Ort und Stelle zu befestigen, wenn sie die Nadel (17) abdeckt, und einen ersten und einen zweiten Eingriffsteil (124, 181; 24, 25; 40, 30, 50) aufweist,
**dadurch gekennzeichnet, dass** der erste und der zweite Eingriffsteil (124, 181; 24, 25; 40, 30, 50) in einer angebrachten Position zusammenwirken, um die Kappe (12) in Position zu befestigen, wenn sie die Nadel (17) abdeckt, und wobei der Anbringmechanismus dazu ausgestaltet ist, sich in eine Freigabeposition zu bewegen, in der der erste und der zweite Eingriffsteil (124, 181; 24, 25; 40, 30, 50) reduziert zusammenwirken, wenn sie über einer Schwellentemperatur liegen, um zum Freilegen der Nadel (17) das Ablösen der Injektorkappe (12) zu ermöglichen.

2. Autoinjektor nach Anspruch 1, wobei der erste und/oder der zweite Eingriffsteil (124, 181; 24, 25; 40, 30, 50) außer Eingriff mit dem jeweils anderen des ersten und zweiten Eingriffsteils (124, 181; 24, 25; 40, 30, 50) in die Freigabeposition vorgespannt ist, wenn sie über der Schwellentemperatur liegen.

3. Autoinjektor nach Anspruch 1 oder Anspruch 2, wobei der erste Eingriffsteil (124, 24, 40) an der Kappe (12) vorgesehen ist und der zweite Eingriffsteil (181, 25, 30) an dem Körper oder der Spritze (18) vorgesehen ist und wobei der erste und der zweite Eingriffsteil (124, 181; 24, 25; 40, 30, 50) zusammenwirken, um die Kappe (12) in der angebrachten Position an dem Körper oder der Spritze (18) zu befestigen, und wobei der erste und/oder der zweite Eingriffsteil (124, 181; 24, 25; 40, 30, 50) in die Freigabeposition vorgespannt ist, wenn der erste und/oder der zweite Eingriffsteil (124, 181; 24, 25; 40, 30, 50) über der Schwellentemperatur liegen, um das Ablösen der Injektorkappe (12) von dem Körper und/oder der Spritze (18) zu ermöglichen.

4. Autoinjektor nach einem der vorhergehenden Ansprüche, wobei die Kappe (12) einen Nadelschutz (123) aufweist, der sich in den Injektorkörper erstreckt, um die Nadel (17) zu umschließen, und wobei der erste Eingriffsteil (124) ein offenes Ende des Nadelschutzes (123) ist und der zweite Eingriffsteil (181) das erste Ende (181) der Spritze (18) ist.

5. Autoinjektor nach Anspruch 4, wobei der Nadelschutz (123) aus einem thermomechanischen Material ausgebildet ist, so dass der Nadelschutz (123) unter der Schwellentemperatur einen ersten Durchmesser hat, bei dem der Nadelschutz (123) an dem ersten Ende (181) der Spritze (18) anliegt, und wobei der Nadelschutz (123) über der Schwellentemperatur in die Freigabeposition vorgespannt ist, in der der Nadelschutz (123) einen zweiten Durchmesser hat, um von dem ersten Ende (181) der Spritze (18) beabstandet zu sein.

6. Autoinjektor nach einem der Ansprüche 1 bis 3, wobei die Kappe (12) eine Außenwand (121) hat, die sich über eine Außenfläche des Körpers erstreckt, und wobei der erste Eingriffsteil (24) ein in der Außenwand (121) ausgebildeter Clip (24) ist und der zweite Eingriffsteil (25) eine in der Außenfläche des Injektorkörpers ausgebildete Öffnung (25) ist.

7. Autoinjektor nach Anspruch 6, wobei der Clip (24) aus einem thermomechanischen Material ausgebildet ist, so dass der Clip (24) unter der Schwellentemperatur in der Öffnung (25) in der Außenfläche des Injektorkörpers angeordnet ist, und wobei der Clip (24) über der Schwellentemperatur von der Außenfläche des Injektorkörpers weg in die Freigabeposition vorgespannt ist, wobei der Clip (24) aus der Öffnung (25) entfernt ist.

8. Autoinjektor nach einem der Ansprüche 1 bis 3, wobei die Kappe (12) eine Außenwand (121) hat, die sich über eine Außenfläche des Körpers erstreckt, und wobei der erste Eingriffsteil (40) eine durch die Außenwand (121) hindurch ausgebildete Öffnung (40) ist und wobei der zweite Eingriffsteil (30, 50) einen in dem Injektorkörper ausgebildeten Clip (30) aufweist.

9. Autoinjektor nach Anspruch 8, wobei der zweite Eingriffsteil (30, 50) ferner eine Auslegerfeder (50) aufweist, die ein festgelegtes Ende, das von einer Innenfläche des Injektorkörpers herabhängt, und ein mechanisch an den Clip (30) gekoppeltes freies Ende hat.

10. Autoinjektor nach Anspruch 9, wobei die Auslegerfeder (50) aus einem thermomechanischen Material ausgebildet ist, so dass die Feder (50) unter der Schwellentemperatur dazu ausgestaltet ist, den Clip (30) so vorzuspannen, dass er in der Öffnung (40) angeordnet ist, und wobei die Feder (50) über der Schwellentemperatur dazu ausgestaltet ist, den Clip (30) in die Freigabeposition vorzuspannen, wobei der Clip (30) aus der Öffnung entfernt ist.

11. Autoinjektor nach einem der Ansprüche 5, 7 oder 10, wobei das thermomechanische Material ein bimetallisches Material ist.

12. Autoinjektor nach einem der Ansprüche 5, 7 oder 10, wobei das thermomechanische Material eine Formgedächtnislegierung ist.

13. Autoinjektorvorrichtung nach einem der vorhergehenden Ansprüche, aufweisend eine Kartusche mit flüssigem Medikament.

## Revendications

1. Dispositif d'auto-injecteur pour administrer un médicament liquide, comprenant :
- un corps d'injecteur,
- une seringue (18) reçue dans le corps d'injecteur,
- une aiguille (17) disposée dans une première extrémité (181) de la seringue (18) pour s'étendre vers une ouverture du corps d'injecteur,
- un capuchon d'injecteur (12) prévu pour entourer l'ouverture et recouvrir l'aiguille, et
- un mécanisme de fixation libérable configuré pour fixer en place le capuchon recouvrant l'aiguille (17) et comprenant des première et deuxième parties d'engagement (124, 181 ; 24, 25 ; 40, 30, 50) ;
**caractérisé en ce que** les première et deuxième parties d'engagement (124, 181 ; 24, 25 ; 40, 30, 50) coopèrent, dans une position fixée, pour fixer en position le capuchon (12) recouvrant l'aiguille (17) et le mécanisme de fixation étant configuré pour se déplacer dans une position de libération dans laquelle les première et deuxième parties d'engagement (124, 181 ; 24, 25 ; 40, 30, 50) coopèrent de manière réduite au-dessus d'une température seuil afin de faciliter le détachement du capuchon d'injecteur (12) pour exposer l'aiguille (17).

2. Auto-injecteur selon la revendication 1, dans lequel au moins l'une parmi les première et deuxième parties d'engagement (124, 181 ; 24, 25 ; 40, 30, 50) est sollicitée hors d'engagement avec l'autre parmi les première et deuxième parties d'engagement (124, 181 ; 24, 25 ; 40, 30, 50) jusque dans la position de libération au-dessus de la température seuil.

3. Auto-injecteur selon la revendication 1 ou la revendication 2, dans lequel la première partie d'engagement (124, 24, 40) est prévue sur le capuchon (12) et la deuxième partie d'engagement (181, 25, 30) est prévue sur le corps ou sur la seringue (18) et dans lequel les première et deuxième parties d'engagement (124, 181 ; 24, 25 ; 40, 30, 50) coopèrent pour fixer le capuchon (12) au corps ou à la seringue (18) dans la position fixée, et dans lequel la première et/ou la deuxième partie d'engagement (124, 181 ; 24, 25 ; 40, 30, 50) est sollicitée dans la position de libération lorsque ladite première et/ou deuxième partie d'engagement (124, 181 ; 24, 25 ; 40, 30, 50) est au-dessus de la température seuil de manière à faciliter le détachement du capuchon d'injecteur (12) du corps et/ou de la seringue (18).

4. Auto-injecteur selon l'une quelconque des revendications précédentes, dans lequel le capuchon (12) comprend un protège-aiguille (123) qui s'étend jusque dans le corps d'injecteur pour entourer l'aiguille (17) et dans lequel la première partie d'engagement (124) est une extrémité ouverte du protège-aiguille (123) et la deuxième partie d'engagement (181) est la première extrémité (181) de la seringue (18).

5. Auto-injecteur selon la revendication 4, dans lequel le protège-aiguille (123) est formé d'un matériau thermomécanique de telle sorte qu'en dessous de la température seuil, le protège-aiguille (123) présente un premier diamètre dans lequel le protège-aiguille (123) bute contre la première extrémité (181) de la seringue (18), et dans lequel, au-dessus de la température seuil, le protège-aiguille (123) est sollicité dans la position de libération dans laquelle le protège-aiguille (123) présente un deuxième diamètre de manière à ce qu'il soit espacé de la première extrémité (181) de la seringue (18).

6. Auto-injecteur selon l'une quelconque des revendications 1 à 3, dans lequel le capuchon (12) présente une paroi extérieure (121) qui s'étend par-dessus une surface extérieure du corps et dans lequel la première partie d'engagement (24) est une pince (24) formée dans la paroi extérieure (121) et la deuxième partie d'engagement (25) est une ouverture (25) formée dans la surface extérieure du corps d'injecteur.

7. Auto-injecteur selon la revendication 6, dans lequel la pince (24) est formée d'un matériau thermomécanique de telle sorte qu'en dessous de la température seuil, la pince (24) soit disposée dans l'ouverture (25) dans la surface extérieure du corps d'injecteur, et dans lequel, au-dessus de la température seuil, la pince (24) est sollicitée à l'écart de la surface extérieure du corps d'injecteur jusque dans la position de libération dans laquelle la pince (24) est retirée de l'ouverture (25).

8. Auto-injecteur selon l'une quelconque des revendications 1 à 3, dans lequel le capuchon (12) présente une paroi extérieure (121) qui s'étend par-dessus une surface extérieure du corps et dans lequel la première partie d'engagement (40) est une ouverture (40) formée à travers la paroi extérieure (121), et dans lequel la deuxième partie d'engagement (30, 50) comprend une pince (30) formée dans le corps d'injecteur.

9. Auto-injecteur selon la revendication 8, dans lequel la deuxième partie d'engagement (30, 50) comprend en outre un ressort en porte-à-faux (50) ayant une extrémité fixe partant d'une surface interne du corps d'injecteur et une extrémité libre accouplée mécaniquement à la pince (30).

10. Auto-injecteur selon la revendication 9, dans lequel le ressort en porte-à-faux (50) est formé d'un matériau thermomécanique de telle sorte qu'en dessous de la température seuil, le ressort (50) soit configuré pour solliciter la pince (30) de manière à ce qu'elle soit disposée dans l'ouverture (40), et dans lequel, au-dessus de la température seuil, le ressort (50) est configuré pour solliciter la pince (30) dans la position de libération dans laquelle la pince (30) est retirée de l'ouverture.

11. Auto-injecteur selon l'une quelconque des revendications 5, 7 ou 10, dans lequel le matériau thermomécanique est un matériau bimétallique.

12. Auto-injecteur selon l'une quelconque des revendications 5, 7 ou 10, dans lequel le matériau thermomécanique est un alliage à mémoire de forme.

13. Dispositif d'auto-injecteur selon l'une quelconque des revendications précédentes, comprenant une cartouche de médicament liquide.
